(19) Europäisches Patentamt
European Patent Office
Office européen des brevets

(11) Veröffentlichungsnummer: **0 077 759**
**B1**

(12) # EUROPÄISCHE PATENTSCHRIFT

(45) Veröffentlichungstag der Patentschrift: **09.12.87**

(51) Int. Cl.⁴: **C 07 D 213/64**, A 01 N 47/34

(21) Anmeldenummer: **82810428.1**

(22) Anmeldetag: **14.10.82**

(54) **Phenylharnstoffe.**

(30) Priorität: **20.10.81 CH 6691/81**
**26.05.82 CH 3231/82**
**03.09.82 CH 5248/82**

(43) Veröffentlichungstag der Anmeldung: **27.04.83 Patentblatt 83/17**

(45) Bekanntmachung des Hinweises auf die Patenterteilung: **09.12.87 Patentblatt 87/50**

(84) Benannte Vertragsstaaten: **AT BE CH DE FR GB IT LI NL**

(56) Entgegenhaltungen:
**EP - A - 0 028 870**
**DE - A - 2 748 636**
**US - A - 4 003 733**

(73) Patentinhaber: **CIBA-GEIGY AG, Klybeckstrasse 141, CH-4002 Basel (CH)**

(72) Erfinder: **Böger, Manfred, Wilhelm Glock-Strasse 14, D-7858 Weil am Rhein 5 (DE)**
Erfinder: **Ehrenfreund, Josef, Dr., Langenhagweg 11, CH-4123 Allschwil (DE)**
Erfinder: **Martin, Pierre, Dr., Meisenweg 38, CH-4310 Rheinfelden (CH)**
Erfinder: **Steiner, Eginhard, Dr., Obere Hofackerstrasse 3, CH-4414 Füllinsdorf (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 077 759 B1

**Beschreibung**

Die vorliegende Erfindung betrifft neue substituierte N-(2-Pyridyloxyphenyl)-N′-benzoylharnstoffe, Verfahren zu ihrer Herstellung und ihre Verwendung in der Schädlingsbekämpfung. Die Erfindung betrifft ferner die zur Herstellung dieser Verbindungen geeigneten neuen Zwischenprodukte sowie Verfahren zur Herstellung der Zwischenprodukte.

Die erfindungsgemässen substituierten N-(2-Pyridyloxyphenyl)-N′-benzoylharnstoffe haben die Formel I

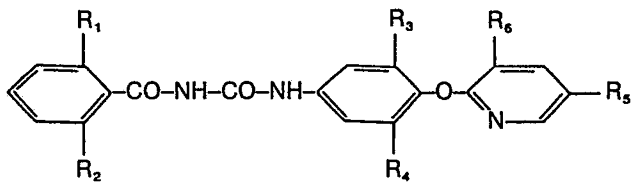

(I),

worin

$R_1$, $R_2$, $R_3$ und $R_4$ unabhängig voneinander Wasserstoff, Methyl oder Halogen;

$R_5$ den Rest $-CHF_2$ oder einen einheitlich oder uneinheitlich mit 1 bis 21 Halogenatomen substituierten $C_2$–$C_{10}$-Alkylrest und

$R_6$ Halogen bedeuten.

Unter Halogen im Rahmen der vorliegenden Erfindung ist vorzugsweise F, Cl und Br zu verstehen, insbesondere F und Cl.

Wegen ihrer Wirkung als Schädlingsbekämpfungsmittel bevorzugt sind Verbindungen der Formel I, die dadurch gekennzeichnet sind, dass $R^5$ den Rest $-CHF_2$ oder einen einheitlich oder uneinheitlich mit 1 bis 5 Fluor- oder Chloratomen substituierten Aethylrest, insbesondere einen der Reste $-CH_2-CF_3$, $-CF_2-CF_2Cl$, $-CF_2-CFCl_2$, $-CCl_2-CCl_3$, $-CF_2-CCl_3$, $-CF_2-CH_3$, $-CCl_2-CH_3$, $-CF_2-CF_3$, $-CH_2-CH_2Cl$, $-CH_2-CHCl_2$ oder $-CH_2-CCl_3$, bedeutet. Wertvoll sind aufgrund ihrer biologischen Wirksamkeit ferner solche Verbindungen der Formel I, worin $R_3$ und $R_4$ unabhängig voneinander Wasserstoff, Methyl oder Chlor bedeuten. Hervorzuheben sind insbesondere Verbindungen der Formel I, worin $R_6$ Chlor bedeutet.

Die Verbindungen der Formel I können nach an sich bekannten Verfahren hergestellt werden (vgl. u.a. die deutschen Offenlegungsschriften Nr. 2 123 236, 2 601 780 und 2 748 636).

So kann man z.B. eine Verbindung der Formel I erhalten durch Umsetzung

a) einer Verbindung der Formel II

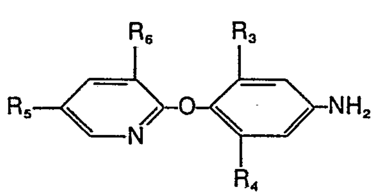

(II)

mit einer Verbindung der Formel III

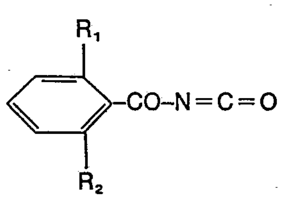

(III)

oder

b) einer Verbindung der Formel IV

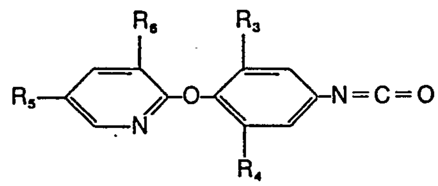

(IV)

mit einer Verbindung der Formel V

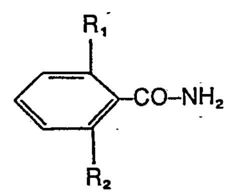

(V).

In den obigen Formeln II, III, IV und V haben die Reste $R_1$, $R_2$, $R_3$, $R_4$, $R_5$ und $R_6$ die unter Formel I vorstehend angegebenen Bedeutungen.

Die erwähnten Verfahren a) und b) können vorzugsweise unter normalem Druck und in Gegenwart eines organischen Lösungs- oder Verdünnungsmittels durchgeführt werden. Als Lösungs- oder Verdünnungsmittel eignen sich z.B. Äther und ätherartige Verbindungen, wie Diäthyläther, Dipropyläther, Dibutyläther, Dioxan, Dimethoxyäthan und Tetrahydrofuran; N,N-dialkylierte Carbonsäureamite; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von — bis 100 °C, vorzugsweise zwischen 15 und 40 °C, gegebenenfalls in Gegenwart einer organischen Base, z.B. Triäthylamin, durchgeführt. Die Durchführung von Verfahren b) erfolgt bei einer Temperatur von 0 bis 120 °C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium.

Die p-Pyridyloxyaniline der Formel II und die p-Pyridyloxyphenylisocyanate der Formel IV sind neue Verbindungen. Diese Verbindungen und ihre Herstellung gehören ebenfalls zum Gegenstand der vorliegenden Erfindung. Man kann die Verbindungen der Formel II durch Umsetzung entsprechend substituierter p-Nitrophenole der Formel VI mit entsprechenden, umsetzungsfähigen Pyridinen der Formel VII und anschliessende Reduktion der Nitrogruppe in den entstehenden Verbindungen der Formel VIII nach einem der üblichen Verfahren erhalten [vgl. z.B. Rec. 21, 271 (1902); J. Am. Soc. 68, 1604 (1964); J. Org. Chem. 11, 378 (1946); Rec. 79, 995 (1970)]:

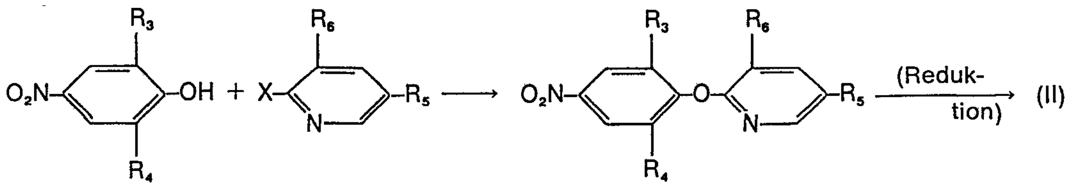


(VI) (VII) (VIII)

In den obigen Formeln VI bis VIII haben $R_3$ bis $R_6$ die vorstehend unter Formel I angegebenen Bedeutungen und X bedeutet Halogen, vorzugsweise Chlor.

Die substituierten Pyridine der Formel VII sind ebenfalls neue Verbindungen, zu denen man mittels einer Ringschlussreaktion durch Umsetzung von Acrylnitril mit einem Aldehyd der Formel $R_5{-}CCl_2{-}CH{=}O$ gegebenenfalls unter Isolierung eines Zwischenproduktes der Formel $N{\equiv}C{-}CHCl{-}CH_2{-}CCl(R_5){-}CH{=}O$ und vorzugsweise in Gegenwart eines Halogenwasserstoffs HX, wobei X Fluor, Chlor oder Brom bedeutet und $R_5$ die unter Formel I angegebene Bedeutung hat, gelangen kann. Dabei kann man die erhaltene Verbindung der Formel VII durch weitere Halogenierung oder Halogenaustausch in der Gruppe $R_5$ im Rahmen der vorstehend unter Formel I angegebenen Bedeutung modifizieren.

Die Pyridyloxyphenylisocyanate der Formel IV sind durch Phosgenisierung der entsprechend substituierten Aniline der Formel II nach allgemein üblichen Arbeitsweisen erhältlich.

Die Ausgangsstoffe der vorstehenden Formeln III und V sind bekannt und lassen sich analog bekannten Verfahren herstellen. So kann man zu den Verbindungen der Formel III wie folgt gelangen (vgl. J. Agr. Food. Chem. 21(3), 348–993, 1973):

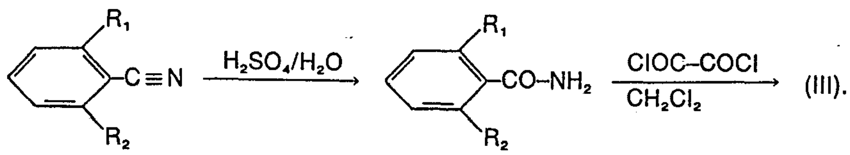


In den obigen Formeln haben $R_1$ und $R_2$ die unter Formel I angegebenen Bedeutungen.

Es ist bekannt, dass bestimmte N-Phenyl-N′-benzoylharnstoffe insektizide Eigenschaften besitzen (vgl. europäische Patentanmeldung 0 016 729, deutsche Offenlegungsschriften 2 123 236, 2 504 982, 2 537 413, 2 601 780 und 2 726 684, die belgischen Patentschriften 832 304, 843 906, 844 066 und 867 046 sowie die US-Patentschrift 4 089 975). Aus der deutschen Offenlegungsschrift 2 748 636 sind bereits substituierte N-(Trifluormethylpyridyloxy)-phenyl-N′-benzoylharnstoffe bekannt, die insektizide Eigenschaften aufweisen.

Überraschenderweise wurde demgegenüber gefunden, dass die erfindungsgemässen Verbindungen der Formel I bei guter Pflanzenverträglichkeit und geringer Warmblütertoxizität eine ausgeprägte Wirksamkeit als Schädlingsbekämpfungsmittel besitzen. Sie eignen sich vor allem zur Bekämpfung von Pflanzen und Tiere befallenden Schädlingen.

Insbesondere eignen sich die Verbindungen der Formel I zur Bekämpfung von Insekten der Ordnungen:

Lepidoptera, Coleoptera, Homoptera, Heteroptera, Diptera, Thyanoptera, Orthoptera, Anoplura, Siphonaptera, Mallophaga, Thysanura, Isoptera, Psocoptera und Hymenoptera.

Die gute insektizide Wirkung der erfindungsgemässen Verbindungen der Formel I entspricht einer Abtötungsrate (Mortalität) von mindestens 50–60% der erwähnten Schadinsekten.

Neben ihrer sehr günstigen Wirkung gegenüber Fliegen, wie z.B. Musca domestica, und Mückenlarven eignen sich Verbindungen der Formel I auch zur Bekämpfung von pflanzenschädigenden Frassinsekten, in Zier- und Nutzpflanzungen, insbesondere in Baumwollkulturen (z.B. gegen Spodoptera littoralis und Heliothis virescens) sowie in Gemüsekulturen (z.B. gegen Leptinotarsa decemlineata und Pieris brassicae). Hervorzuheben ist besonders die ovizide bzw. ovolarvizide Wirkung von Verbindungen der Formel I. Werden Verbindungen der Formel I von adulten Insekten mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I eignen sich weiterhin zur Bekämpfung von Ektoparasiten an Haus- und Nutztieren, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die den Wirkstoff der Formel I, bzw. Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Äther und Ester, wie Aethanol, Aethylenglykol, Aethylenglykolmonomethyl- oder äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxydiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach der Art des zu formulierenden Wirkstoffes der Formel I oder der Kombinationen dieser Wirkstoffe und andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Talgöl gewonnen werden können. Ferner sind auch die Fettsäuremethyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxy-Addukten. Die sulfonierten Benzimidazolderivate enthalten vorzugsweise 2 Sulfonsäuregruppen und einen Fettsäurerest mit etwa 8–22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4–14)-Aethylenoxyd-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien
Nonylphenolpolyäthoxyäthanole,
Ricinusölpolyglykoläther,

Polypropylen-Polyäthylenoxydaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioelat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyl-di-(2-chloräthyl)-äthylammoniumbromid.

Die in der Formulierungstechnik gebräuchlichen Tenside sind u. a. in folgenden Publikationen beschrieben:

«Mc Cutcheon's Detergents and Emulsifiers Annual» MC Publishing Corp., Ringwood, New Jersey, 1979.

Sisely and Wood, «Encyclopedia of Surface Active Agents», Chemical Publishing Co, Inc. New York (1979).

Die pestiziden Zubereitungen enthalten in der Regel 0,1 bis 99%, insbesondere 0,1 bis 95%, Wirkstoff der Formel I oder Kombinationen dieser Wirkstoffe mit andern Insektiziden oder Akariziden, 1 bis 99,9% eines festen oder flüssigen Zusatzstoffes und 0 bis 25%, insbesondere 0,1 bis 20%, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I oder Kombinationen dieser Wirkstoffe mit andern Insektiziden oder Akariziden (% = Gewichtsprozent)

| 1. Emulsions-Konzentrate | a) | b) | c) |
|---|---|---|---|
| Wirkstoff resp. Wirkstoffkombination | 25% | 40% | 50% |
| Ca-Dodecylbenzolsulfonat | 5% | 8% | 6% |
| Ricinusölpolyäthylenglykoläther (36 Mol AeO) | 5% | – | – |
| Tributylphenolpolyäthylenglykoläther (30 Mol AeO) | – | 12% | 4% |
| Cyclohexanon | – | 15% | 20% |
| Xylolgemisch | 65% | 25% | 20% |

Aus solchen Konzentraten können durch Verdünnen mit Wasser Emulsionen jeder gewünschten Konzentrationen hergestelt werden.

| 2. Lösungen | a) | b) | c) | d) |
|---|---|---|---|---|
| Wirkstoff resp. Wirkstoffkombination | 80% | 10% | 5% | 95% |
| Aethylenglykolmonomethyläther | 20% | – | – | – |
| P-lyäthylenglykol MG 400 | – | 70% | – | – |
| N-Methyl-2-pyrrolidon | – | 20% | – | – |
| Epoxydiertes Kokosnussöl | – | – | 1% | 5% |
| Benzin (Siedegrenzen 160–190° C) | – | – | 94% | – |

Die Lösung sind zur Anwendung in Form kleinster Tropfen geeignet.

| 3. Granulate | a) | b) |
|---|---|---|
| Wirkstoff resp. Wirkstoffkombination | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsittel anschliessend im Vakuum abgedampft.

| 4. Stäubemittel | a) | b) |
|---|---|---|
| Wirkstoff resp. Wirkstoffkombination | 2% | 5% |
| Hochdisperse Kieselsäure | 1% | 5% |
| Talkum | 97% | – |
| Kaolin | – | 90% |

Durch inniges Vermischen der Trägerstoffe mit dem Wirkstoff erhält man gebrauchsfertige Stäubemittel.

Formulierungsbeispiele für feste Wirkstoffe der Formel I resp. Kombinationen dieser Wirkstoffe mit andern Insektiziden oder Akariziden (% = Gewichtsprozent)

| 5. Spritzpulver | a) | b) | c) |
|---|---|---|---|
| Wirkstoff oder Wirkstoffkombination | 25% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyäthylenglykoläther (7-8 Mol AeO) | – | 2% | – |
| Hochdisperse Kieselsäure | 5% | 10% | 10% |
| Kaolin | 62% | 27% | – |

Der Wirkstoff oder die Wirkstoffkombination werden mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

6. Emulsions-Konzentrat

| | |
|---|---|
| Wirkstoff oder Wirkstoffkombination | 10% |
| Octylphenolpolyäthylenglykoläther (4–5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können auch Verdünnen mit Wasser Emulsionen jeder gewünschten Konzentration hergestellt werden.

| 7. Stäubemittel | a) | b) |
|---|---|---|
| Wirkstoff oder Wirkstoffkombination | 5% | 8% |
| Talkum | 95% | – |
| Kaolin | – | 92% |

Man erhält anwendungsfertige Stäubemittel, indem der Wirkstoff mit dem Träger vermischt und auf einer geeigneten Mühle vermahlen wird.

8. Extruder-Granulat

| | |
|---|---|
| Wirkstoff oder Wirkstoffkombination | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert, granuliert und anschliessend im Luftstrom getrocken.

9. Umhüllungs-Granulat

| | |
|---|---|
| Wirkstoff oder Wirkstoffkombination | 3% |
| Polyäthylenglykol (MG 200) | 3% |
| Kaolin | 94% |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff oder Wirkstoffkombination | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1:

Herstellung der Ausgangsverbindung 3-Chlor-4-[3-chlor-5-(1'-difluor-2'-dichlorfluoräthyl)-2-pyridyloxy]-anilin

Es werden 1,3 g gepulvertes Kaliumhydroxid mit 10 ml Dimethylsulfoxid vermischt. Nach Zugabe von 2,95 g 2-Chlor-4-nitrophenol wird das Gemisch 1 Stunde bei 100 °C gerührt. Das Gemisch wird dann auf 50 °C abgekühlt. Danach werden 5,1 g

2,3-Dichlor-5-(1′-difluor-2′-dichlorfluoräthyl)-pyridin

gelöst in 5 ml Dimethylsulfoxid hinzugetropft und unter einer Stickstoffatmosphäre wird 5 Stunden bei 120 °C gerührt. Das Reaktionsgemisch wird auf Eiswasser gegossen, mit Toluol extrahiert, die Toluolphase abgetrennt, mit Wasser gewaschen, getrocknet und eingedampft. Man erhält das

3-Chlor-4-[3-chlor-5-(1′-difluor-2′-dichlorfluoräthyl)-2-pyridyloxy]-nitrobenzol

als weisses Pulver vom Schmelzpunkt 76–78 °C.

5,6 g 3-Chlor-4-[3-chlor-5-(1′-difluor-2′-dichlorfluoräthyl)-2-pyridyloxy]-nitrobenzol

werden mit 15 ml konz. Chlorwasserstoffsäure vermischt. Bei 70–75 °C werden 13,1 g Zinndichlorid ($SnCl_2 \cdot 2H_2O$) in 20 ml konz. Chlorwasserstoffsäure tropfenweise zugegeben. Das Gemisch wird 4 Stunden bei ca. 100 °C gerührt. Danach wird das Reaktionsgemisch auf Eis gegossen, mit Natronlauge (50 Gew.% NaOH) alkalisch gestellt, mit Dichlormethan extrahiert, der erhaltene Extrakt neutral gewaschen, getrocknet, stark eingeengt und über Kieselgel filtriert. Das erhaltene Filtrat wird eingedampft und man erhält das

3-Chlor-4-[3-chlor-5′-(1′-difluor-2′-dichlorfluoräthyl)-2-pyridyloxy]-anilin

als helles Öl, $n_{21}^{D}$ = 1,5729.

In analoger Weise werden auch die folgenden Ausgangsverbindungen der Formel II hergestellt

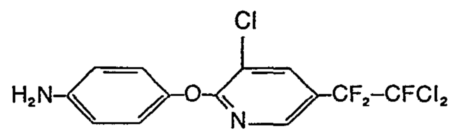

Smp. 87–88 °C

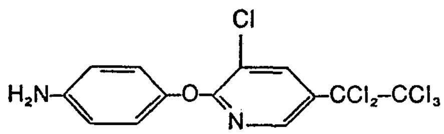

Smp. 144–146 °C

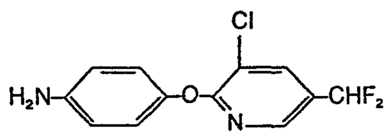

Smp. 58–60 °C

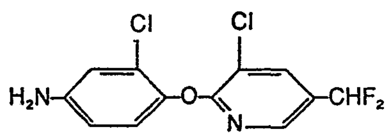

Smp. 89–90 °C

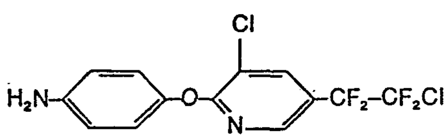

Smp. 84-86 °C

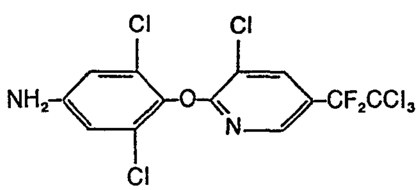

Smp. 120–122 °C

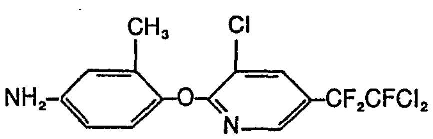

Smp. 47–48 °C

Herstellung von N[1]–3-Chlor-4-[3-chlor-5-(1′-difluor-2′-dichlorfluoräthyl)2-pyridyloxy]-phenyl-N[2]–2-chlorbenzoylharnstoff:

Es werden 2,25 g des erhaltenen

3-Chlor-4-[3-chlor-5-(1′-difluor-2′-dichlorfluoräthyl)-2-pyridyloxy]-anilins

in 20 ml absolutem Toluol gelöst und unter Ausschluss von Feuchtigkeit tropfenweise mit 1 g 2-Chlorbenzoylisocyanat gelöst in 10 ml Toluol versetzt. Man lässt die exotherme Reaktion abklingen. Der nach einiger Zeit ausgefallene kristalline Niederschlag wird abgesaugt, mit wenig Hexan gewaschen und getrocknet. Man erhält

N[1]–3-Chlor-4-[3-chlor-5(1′-difluor-2′-dichlorfluoräthyl)-2-pyridyloxy]-phenyl-N[2]–2-chlorbenzoylharnstoff

vom Schmelzpunkt 225–227 °C (Verbindung Nr. 1).

Analog den vorstehend beschriebenen Arbeitsweisen wurden die folgenden Verbindungen der Formel I hergestellt:

| Verb. Nr. | $R_1$ | $R_2$ | $R_3$ | $R_4$ | $R_5$ | $R_6$ | Schmelzpunkt [°C] |
|---|---|---|---|---|---|---|---|
| 1 | Cl | H | Cl | H | $-CF_2-CFCl_2$ | Cl | 225-227 |
| 2 | F | F | H | H | $-CF_2-CFCl_2$ | Cl | 198-200 |
| 3 | Cl | H | H | H | $-CF_2-CFCl_2$ | Cl | 186-188 |
| 4 | F | F | Cl | H | $-CF_2-CFCl_2$ | Cl | 203-205 |
| 5 | F | F | H | H | $-CCl_2-CCl_3$ | Cl | 229-231 |
| 6 | F | F | H | H | $-CHF_2$ | Cl | 170-172 |
| 7 | F | F | Cl | H | $-CHF_2$ | Cl | 219-222 |
| 8 | F | F | H | H | $-CF_2-CF_2Cl$ | Cl | 188-190 |
| 9 | Cl | H | H | H | $-CF_2-CF_2Cl$ | Cl | 191-193 |
| 10 | F | F | Cl | Cl | $-CF_2-CF_2Cl$ | Cl | 227-229 |
| 11 | Cl | H | Cl | Cl | $-CF_2-CF_2Cl$ | Cl | 211-213 |
| 12 | F | F | Cl | Cl | $-CF_2-CCl_3$ | Cl | 234-236 |
| 13 | F | F | Cl | Cl | $CF_2-CFCl_2$ | Cl | 231-233 |
| 14 | Cl | H | Cl | Cl | $CF_2-CFCl_2$ | Cl | 220-222 |
| 15 | Br | H | Cl | Cl | $CF_2-CCl_3$ | Cl | 221-223 |
| 16 | $CH_3$ | H | Cl | Cl | $CF_2-CCl_3$ | Cl | 236-238 |
| 17 | F | F | $CH_3$ | H | $CF_2-CFCl_2$ | Cl | 190-192 |
| 18 | Cl | H | $CH_3$ | H | $CF_2-CF_2Cl$ | Cl | 195-196 |
| 19 | H | H | Cl | Cl | $CF_2-CF_2Cl$ | Cl | $>240$ |

Ferner sind entsprechend den vorstehend beschriebenen Arbeitsweisen die folgenden Verbindungen der Formel I erhältlich:

| Verb. Nr. | $R_1$ | $R_2$ | $R_3$ | $R_4$ | $R_5$ | $R_6$ |
|---|---|---|---|---|---|---|
| 20 | Cl | H | H | H | $-CCl_2-CCl_3$ | Cl |
| 21 | F | Cl | Cl | Cl | $-CF_2-CFCl_2$ | Cl |
| 22 | $CH_3$ | H | Cl | Cl | $-CF_2-CFCl_2$ | Cl |
| 23 | F | F | Cl | Cl | $-CF_2-CH_3$ | Cl |
| 24 | Cl | H | Cl | Cl | $-CF_2-CH_3$ | Cl |
| 25 | F | Cl | Cl | Cl | $-CF_2-CH_3$ | Cl |
| 26 | F | F | Cl | Cl | $-CF_2-CF_3$ | Cl |
| 27 | Cl | H | Cl | Cl | $-CF_2-CF_3$ | Cl |
| 28 | F | Cl | Cl | Cl | $-CF_2-CF_3$ | Cl |
| 29 | F | F | Cl | Cl | $-CCl_2-CH_3$ | Cl |
| 30 | Cl | H | Cl | Cl | $-CCl_2-CH_3$ | Cl |
| 31 | F | Cl | Cl | Cl | $-CCl_2-CH_3$ | Cl |
| 32 | F | F | Br | Br | $-CF_2-CHFCl$ | Cl |
| 33 | Cl | H | Br | Br | $-CF_2-CHFCl$ | Cl |
| 34 | F | F | Cl | H | $-CCl_2-CCl_3$ | Cl |
| 35 | Cl | H | Cl | H | $-CCl_2-CCl_3$ | Cl |
| 36 | F | F | Cl | Cl | $-CF_2-CFCl_2$ | F |
| 37 | F | F | Cl | Cl | $-CF_2-CFCl_2$ | Br |
| 38 | H | H | Cl | Cl | $-CF_2-CFCl_2$ | Cl |
| 39 | Br | Br | Cl | Cl | $-CF_2-CFCl_2$ | Cl |
| 40 | $CH_3$ | $CH_3$ | Cl | Cl | $-CF_2-CFCl_2$ | Cl |
| 41 | F | F | F | F | $-CF_2-CFCl_2$ | Cl |

Beispiel 2:
Wirkung gegen Musca domestica

Je 50 g frisch zubereitetes CSMA-Nährsubstrat für Maden wird in Becher eingewogen. Von einer 1 Gew.%igen acetonischen Lösung des betreffenden Wirkstoffes wird eine bestimmte Menge auf das in den Bechern befindliche Nährsubstrat pipettiert. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahlk der aus den Puppen geschlüpften Fliegen bestimmt.

Verbindungen der Formel I gemäss Beispiel 1 zeigen gute Wirkung im obigen Test.

Beispiel 3:
Wirkung gegen Lucilia sericata

Zu 9 ml eines Zuchtmediums wird bei 50 °C 1 ml einer 0,5% Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 4:
Wirkung gegen Aedes aëgypti

Auf die Oberfläche von 150 ml Wasser, das sich in einem Behälter befindet, wird so viel einer 0,1%igen acetonischen Lösung des Wirkstoffes pipettiert, dass eine Konzentration von 12,5 ppm erhalten wird. Nach Verdunsten des Acetons wird der Behälter mit 30–40 2-tägigen Aëdes-Larven beschickt. Nach 2 und 7 Tagen wird die Mortalität geprüft.

Beispiel 5:
Insektizide Frassgift-Wirkung

Baumwollpflanzen (ca. 20 cm hoch) werden mit wässrigen Wirkstoffemulsionen (erhalten aus einem 10%igen emulgierbaren Konzentrat) besprüht, wobei die Wirkstoffemulsion 0,75, 3,0, 12,5, 50, 100 und 400 ppm der zu prüfenden Verbindung enthalten.

Nach dem Antrocknen des Belages werden die Baumwollpflanzen je mit Spodoptera littoralis- und Heliothis virescens-Larven im dritten larvalen Stadium besetzt. Der Versuch wird bei 24 °C und 60% relativer Luftfeuchtigkeit durchgeführt. In Abständen von jeweils 24 Stunden werden Mortalität sowie Entwicklungs- und Häutungsstörungen der angesetzten Larven bestimmt.

Beispiel 6:
Wirkung auf Spodoptera litteralis und Heliothis virescens (Larven und Eier)

Es werden drei in Töpfen gezogene Baumwollpflanzen von ca. 15–20 cm Höhe mit einer sprühfähigen flüssigen Zubereitung des zu prüfenden Wirkstoffes behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;

b) 20 Larven von Spodoptera littoralis oder Heliothis virescens des dritten larvalen Stadiums;

c) zwei Eispiegeln von Spodoptera littoralis oder Heliotis virescens (dazu werden je 2 Blätter einer Pflanze in einem beidseitig mit Gaze verschlossenen Plexiglaszylinder eingeschlossen); zwei Eispiegel von Spodoptera oder ein Teil eines Baumwollblattes mit darauf abgelegten Eiern von Heliothis werden zu den eingeschlossenen Blättern gegeben.

Nach 4 und 5 Tagen erfolgt die Auswertung gegenüber unbehandelten Kontrollen unter Berücksichtigung folgender Kriterien:

a) Anzahl der noch lebenden Larven,
b) larvale Entwicklungs- und Häutungshemmung,
c) Frassschaden (Schabfrass und Lochfrass),
d) Schlupfrate (Anzahl der aus den Eiern geschlüpften Larven).

Verbindungen der Formel I gemäss Beispiel 1 zeigen gute Gesamt-Wirksamkeit in obigem Test.

Beispiel 7:
Ovizide Wirkung auf Spodoptera littoralis

Auf Filterpapier abgelegte Eier von Spodoptera littoralis werden aus dem Papier ausgeschnitten und in eine 0,05 Gew.%ige Lösung des Wirkstoffes in einem Acezon-Wasser-Gemisch (1:1) getaucht. Die so behandelten Eiablagen werden dann aus diesem Gemisch herausgenommen und bei 28 °C und 60% relativer Feuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen der Formel I gemäss Beispiel 1 zeigen gute Wirkung im obigen Test.

Beispiel 8:
Ovizide Wirkung auf Epilachna varivestis

Es werden 20 Gew.% Wirkstoff, 70 Gew.% Xylol und 10 Gew.% einer Mischung aus einem Reaktionsprodukt eines Alkylphenols mit Aethylenoxyd und Calcium-dodecylbenzolsulfonat miteinander vermischt. Aus diesem Konzentrat werden wässrige Emulsionen enthaltend 800 und 1600 ppm Wirkstoff hergestellt.

Jeweils ca. 100 auf Blätter von Phaseolus vulgaris frisch abgelegte Eier von Epilachna varivestis (mexikanischer Bohnenkäfer) werden mit den oben beschriebenen wässrigen Emulsionen (Konzentration 800 bzw. 1600 ppm Wirkstoff) angefeuchtet und leicht getrocknet.

In einem gelüfteten Gefäss werden die behandelten Gelege solange gehalten, bis die gleichzeitig angesetzten unbehandelten Kontrollen geschlüpft sind. Unter einem Binocular erfolgt Auswertung hinsichtlich der erzielten prozentualen Abtötung.

Verbindungen der Formel I gemäss Beispiel 1 zeigen gute Wirkung in obigem Test.

Beispiel 9:
Ovizide Wirkung auf Heliothis virescens und Leptinotarsa decemlineata

Entsprechende Mengenanteile einer benetzbaren pulverförmigen Formulierung, enthaltend 25 Gew.% des zu prüfenden Wirkstoffes, werden mit jeweils soviel Wasser vermischt, dass sich wässrige Emulsionen von ansteigender Wirkstoffkonzentration ergeben.

In diese wirkstoffhaltigen Emulsionen werden eintätige Eigelege von Heliothis auf Cellophan® bzw. Eigelege von Leptinotarsa auf Kartoffelblättern während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt. Zur Auswertung wird die zur 100%igen Abtötung der Eier erforderliche minimale Wirkstoffkonzentration bestimmt.

Verbindungen der Formel I gemäss Beispiel 1 zeigen gute Wirkung in obigem Test.

Beispiel 10:
Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung enthaltend 400 ppm des zu prüfenden Wirkstoffes eingetaucht. Nach dem Antrocknen der Lösung werden die Eier in Petrischalen ausgelegt und bei einer Temperatur von 28 °C belassen. Nach 6 Tagen wird der prozentuale Schlupf aus den behandelten Eiern bewertet.

Verbindungen der Formel I gemäss Beispiel 1 zeigen gute Wirkung in obigem Test.

Beispiel 11:
Reproduktions-Beeinflussung von Anthonomus grandis

Adulte Anthonomus grandis, die nach dem Schlupf nicht älter als 24 Stunden sind, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 1,0 Gew.% des zu prüfenden Wirkstoffes, eingetaucht.

Nachdem die Käfer wieder trocken waren, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäss Beispiel 1 zeigen eine gute reproduktionsreduzierende Wirkung im obigen Test.

Beispiel:12
Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 12,5, 50 und 100 ppm des zu prüfenden Wirkstoffes besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Öffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25 °C und etwa 60% relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Biologische Ergebnisse

Die nachstehende Tabelle zeigt Ergebnisse biologischer Prüfungen erfindungsgemässer Verbindungen auf der Basis obiger biologischer Beispiele. Die Auswertung der Versuche erfolgte anhand der erhaltenen %-Mortalität unter Verwendung des folgenden Bewertungs-Index:

A: 80–100% Mortalität bei einer Konzentration von 0,75 ppm der geprüften Verbindung.
B: 80–100% Mortalität bei einer Konzentration von 3,0 ppm der geprüften Verbindung.
C: 80–100% Mortalität bei einer Konzentration von 12,5 ppm der geprüften Verbindung.
D: 80–100% Mortalität bei einer Konzentration von 50 ppm der geprüften Verbindung.
E: 80–100% Mortalität bei einer Konzentration von 100 ppm der geprüften Verbindung.
F: 80–100% Mortalität bei einer Konzentration von 400 ppm der geprüften Verbindung.

| Verb. Nr. | pestizide Wirksamkeit Aëdes-Larven (Beispiel 4) | Spodoptera-Larven (Beispiel 5) | Heliothis-Larven (Beispiel 5) | Anthonomus (Beispiel 11) |
|---|---|---|---|---|
| 1 | – | D | D | – |
| 2 | C | A | B | – |
| 3 | C | C | E | – |
| 4 | C | B | B | – |
| 5 | C | D | E | – |
| 6 | C | F | – | – |
| 7 | – | D | – | D |
| 8 | – | B | C | C |
| 9 | – | C | D | E |
| 10 | – | C | D | E |
| 11 | – | C | C | E |
| 12 | – | E | E | – |

**Patentansprüche für die Vertragsstaaten BE, CH, DE, FR, GB, IT, LI, NL.**

1. Verbindungen der Formel I

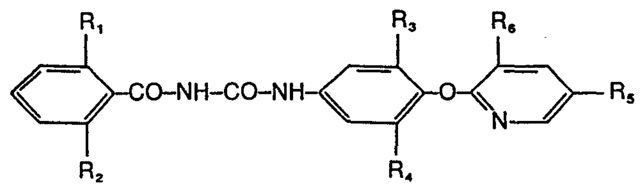

(I),

worin

$R_1$, $R_2$, $R_3$ und $R_4$ unabhängig voneinander Wasserstoff, Methyl oder Halogen;

$R_5$ den Rest $-CHF_2$ oder einen einheitlich oder uneinheitlich mit 1 bis 21 Halogenatomen substituierten $C_2$–$C_{10}$-Alkylrest und

$R_6$ Halogen bedeuten.

2. Verbindungen der Formel I gemäss Anspruch 1, dadurch gekennzeichnet, dass

$R_1$, $R_2$, $R_3$ und $R_4$ unabhängig voneinander Wasserstoff, Methyl, Fluor, Chlor oder Brom;

$R_5$ den Rest $-CHF_2$ oder einen mit 1 bis 21 Fluor-, Chlor- oder Bromatomen substituierten $C_2$–$C_{10}$-Alkylrest und

$R_6$ Fluor, Chlor oder Brom bedeuten.

3. Verbindungen der Formel I gemäss Anspruch 2, dadurch gekennzeichnet, dass $R_5$ den Rest $-CHF_2$ oder einen einheitlich oder uneinheitlich mit 1 bis 5 Fluor- oder Chloratomen substituierten Aethylrest bedeutet.

4. Verbindungen der Formel I gemäss Anspruch 3, dadurch gekennzeichnet, dass $R_5$ einen der Reste $-CH_2-CF_3$, $-CF_2-CF_2Cl$, $-CF_2CFCl_2$, $-CCl_2-CCl_3$, $-CF_2-CCl_3$, $-CF_2-CH_3$, $-CCl_2-CH_3$, $-CF_2-CF_3$, $-CH_2-CH_2Cl$, $-CH_2-CHCl_2$ oder $-CH_2-CCl_3$ bedeutet.

5. Verbindungen der Formel I gemäss Anspruch 1 bis 5, dadurch gekennzeichnet, dass $R_3$ und $R_4$ unabhängig voneinander Wasserstoff, Methyl oder Chlor bedeuten.

6. Verbindung der Formel I gemäss Anspruch 1 bis 6, dadurch gekennzeichnet, dass $R_6$ Chlor bedeutet.

7. Verbindung gemäss Anspruch 4 der Formel

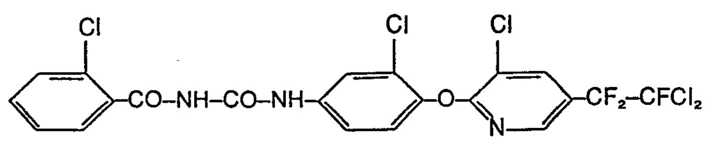

.

8. Verbindung gemäss Anspruch 4 der Formel

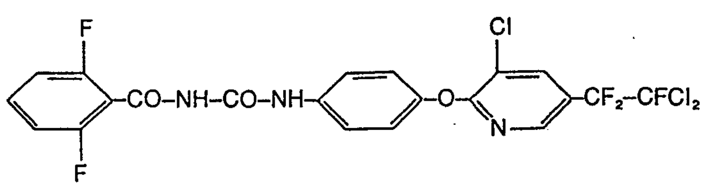

.

9. Verbindung gemäss Anspruch 4 der Formel

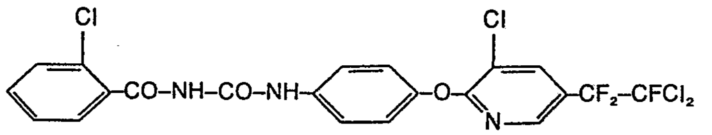

10. Verbindung gemäss Anspruch 4 der Formel

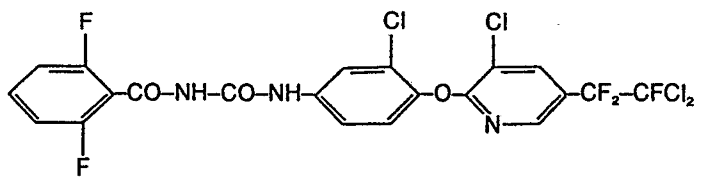

11. Verbindung gemäss Anspruch 4 der Formel

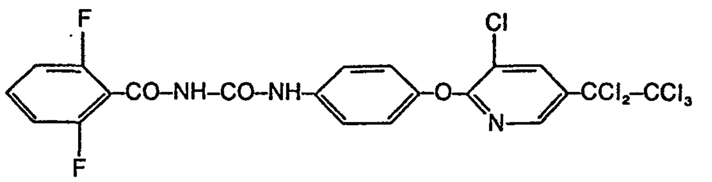

12. Verbindung gemäss Anspruch 4 der Formel

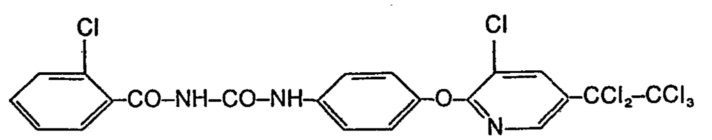

13. Verbindung gemäss Anspruch 3 der Formel

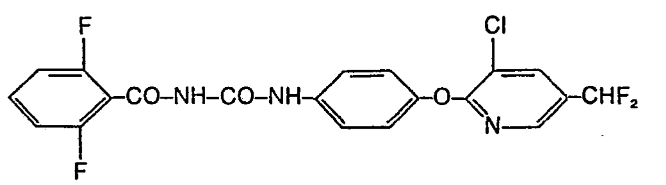

14. Verbindund gemäss Anspruch 3 der Formel

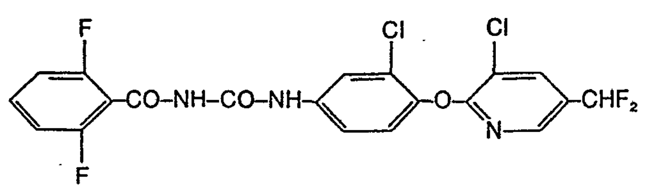

15. Verbindung gemäss Anspruch 4 der Formel

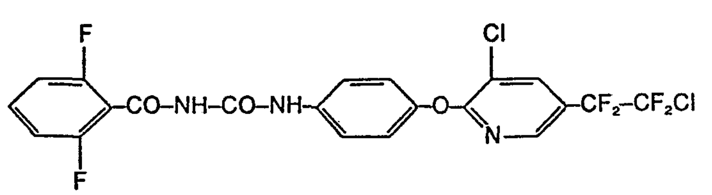

16. Verbindung gemäss Anspruch 4 der Formel

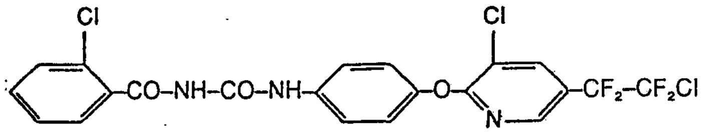

.

17. Verbindung gemäss Anspruch 4 der Formel

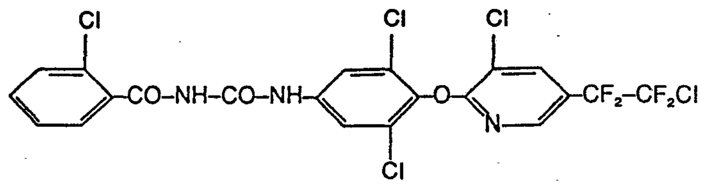

.

18. Verbindung gemäss Anspruch 4 der Formel

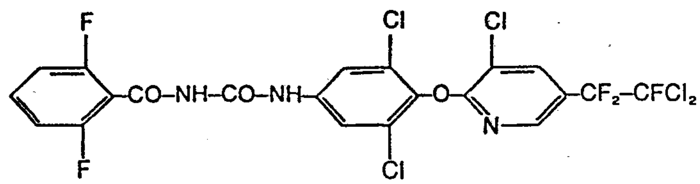

.

19. Verfahren zur Herstellung einer Verbindung gemäss den Ansprüchen 1 bis 18, dadurch gekennzeichnet, dass man

a) eine Verbindung der Formel II

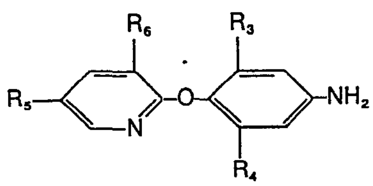

(II)

mit einer Verbindung der Formel III

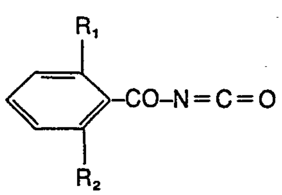

(III)

oder

b) eine Verbindung der Formel IV

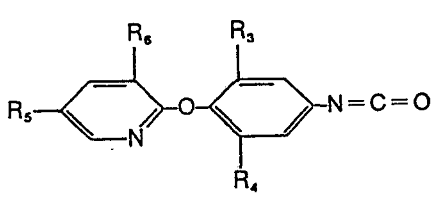

(IV)

mit einer Verbindung der Formel V

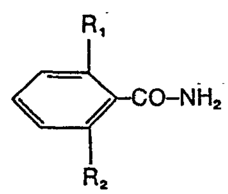

(V).

umsetzt, wobei in den Formeln II bis V die Reste $R_1$ bis $R_6$ die in den Ansprüchen 1 bis 6 angegebenen Bedeutungen haben.

20. Schädlingsbekämpfungsmittel, welches als aktive Komponente eine Verbindung gemäss den Ansprüchen 1 bis 19 zusammen mit geeigneten Trägern und/oder anderen Zuschlagstoffen enthält.

21. Verwendung von Verbindungen gemäss den Ansprüchen 1 bis 18 zur Bekämpfung von Schädlingen, vorzugsweise von Insekten und Vertretern der Ordnung Akarina.

22. Verwendung gemäss Anspruch 21 als Larvizide und Ovizide zur Bekämpfung pflanzenschädigender Insekten.

23. Verbindung der Formel II

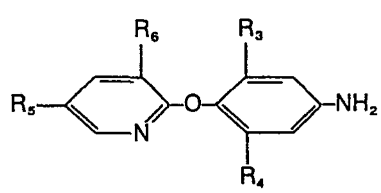

(II)

worin $R_3$ bis $R_6$ die in den Ansprüchen 1 bis 6 angegebenen Bedeutungen haben.

24. Verbindung der Formel IV

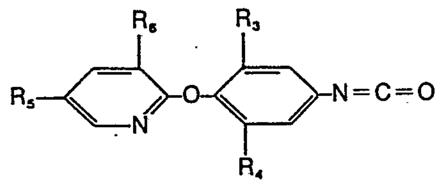

(IV)

worin $R_3$ bis $R_6$ in den Ansprüchen 1 bis 6 angegebenen Bedeutungen haben.

25. Verfahren zur Herstellung einer Verbindung der Formel II gemäss Anspruch 23, dadurch gekennzeichnet, dass man eine Verbindung der Formel VI

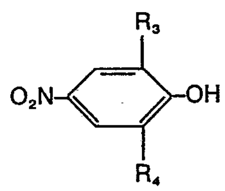

(VI)

mit einer Verbindung der Formel VII

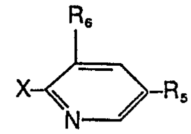

(VII)

umsetzt und die erhaltene Verbindung der Formel VIII

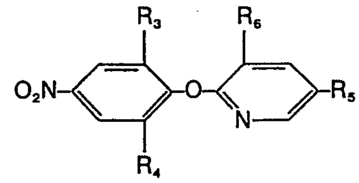

(VIII)

reduziert, wobei in den Formeln VI bis VIII $R_3$ bis $R_6$ die in den Ansprüchen 1 bis 6 angegebenen Bedeutungen haben und X Halogen, vorzugsweise Chlor, bedeutet.

**Patentansprüche für den Vertragsstaat AT**

1. Verfahren zur Herstellung einer Verbindung der Formel I,

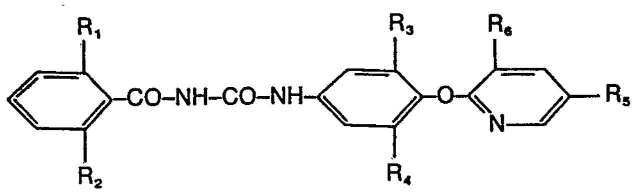

(I),

worin

$R_1$, $R_2$, $R_3$ und $R_4$ unabhängig voneinander Wasserstoff, Methyl oder Halogen;

$R_5$ den Rest $-CHF_2$ oder einen einheitlich oder uneinheitlich mit 1 bis 21 Halogenatomen substituierten $C_2$–$C_{10}$-Alkylrest und

$R_6$ Halogen bedeuten,

dadurch gekennzeichnet, dass man

a) eine Verbindung der Formel II

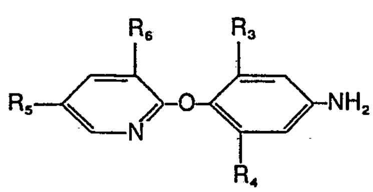

(II)

mit einer Verbindung der Formel III

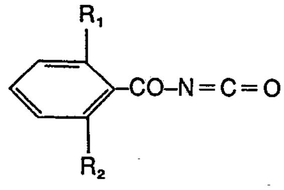

(III)

oder

b) eine Verbindung der Formel IV

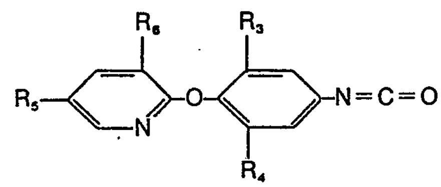

(IV)

mit einer Verbindung der Formel V

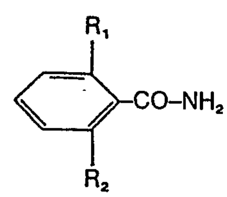

(V)

umsetzt, wobei in den Formeln II bis V die Reste $R_1$ bis $R_6$ die oben angegebenen Bedeutungen haben.

2. Verfahren gemäss Anspruch 1 zur Herstellung einer Verbindung der Formel I, dadurch gekennzeichnet, dass

$R_1$, $R_2$, $R_3$ und $R_4$ unabhängig voneinander Wasserstoff, Methyl, Fluor, Chlor oder Brom;

$R_5$ den Rest $-CHF_2$ oder einen mit 1 bis 21 Fluor-, Chlor- oder Bromatomen substituierten $C_2$–$C_{10}$-Alkylrest und

$R_6$ Fluor, Chlor oder Brom bedeuten.

3. Verfahren gemäss Anspruch 2 zur Herstellung einer Verbindung der Formel I, dadurch gekennzeichnet, dass $R_5$ den Rest $-CHF_2$ oder einen einheitlich oder uneinheitlich mit 1 bis 5 Fluor- oder Chloratomen substituierten Aethylrest bedeutet.

4. Verfahren gemäss Anspruch 3 zur Herstellung einer Verbindung der Formel I, dadurch gekennzeichnet, dass $R_5$ einen der Reste $-CH_2-CF_3$, $-CF_2-CF_2Cl$, $-CF_2CFCl_2$, $-CCl_2-CCl_3$, $-CF_2-CCl_3$, $-CF_2-CH_3$, $-CCl_2-CH_3$, $-CF_2-CF_3$, $-CH_2-CH_2Cl$, $-CH_2-CHCl_2$ oder $-CH_2-CCl_3$ bedeutet.

5. Verfahren gemäss Anspruch 1 bis 4 zur Herstellung einer Verbindung der Formel I, dadurch gekennzeichnet, dass $R_3$ und $R_4$ unabhängig voneinander Wasserstoff, Methyl oder Chlor bedeuten.

6. Verfahren gemäss Anspruch 1 bis 5 zur Herstellung einer Verbindung der Formel I, dadurch gekennzeichnet, dass $R_6$ Chlor bedeutet.

7. Verfahren gemäss Anspruch 4 zur Herstellung der Verbindung der Formel

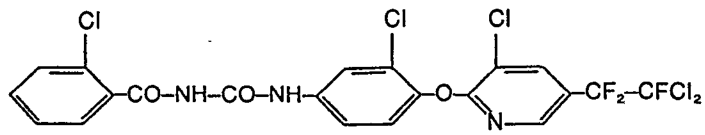 .

8. Verfahren gemäss Anspruch 4 zur Herstellung der Verbindung der Formel

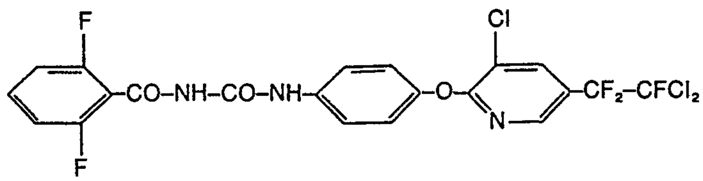 .

9. Verfahren gemäss Anspruch 4 zur Herstellung der Verbindung der Formel

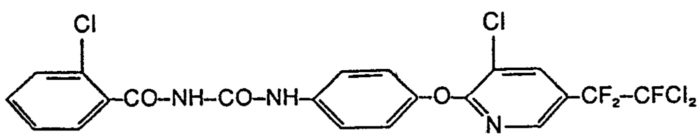 .

10. Verfahren gemäss Anspruch 4 zur Herstellung der Verbindung der Formel

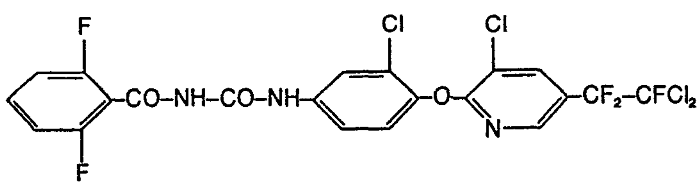 .

11. Verfahren gemäss Anspruch 4 zur Herstellung der Verbindung der Formel

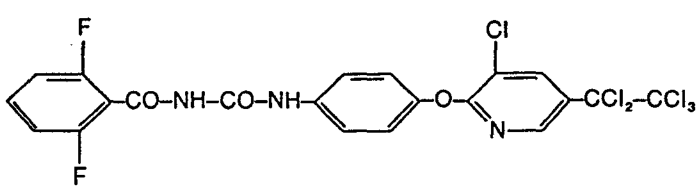 .

12. Verfahren gemäss Anspruch 4 zur Herstellung der Verbindung der Formel

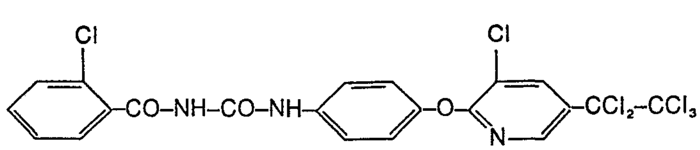 .

13. Verfahren gemäss Anspruch 3 zur Herstellung der Verbindung der Formel

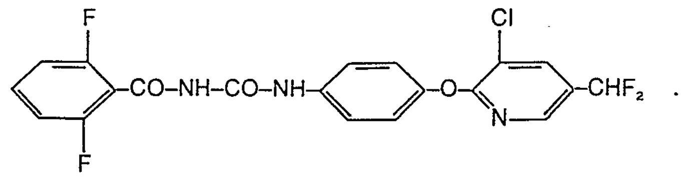

14. Verfahren gemäss Anspruch 3 zur Herstellung der Verbindung der Formel

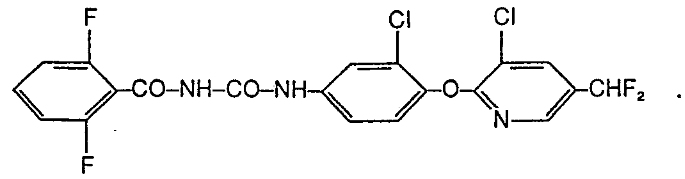

15. Verfahren gemäss Anspruch 4 zur Herstellung der Verbindung der Formel

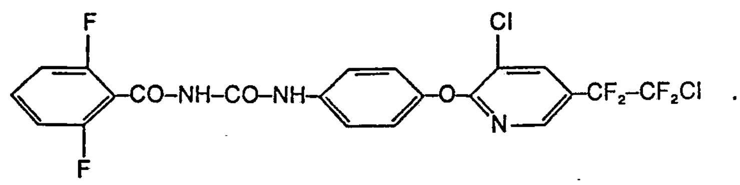

16. Verfahren gemäss Anspruch 4 zur Herstellung der Verbindung der Formel

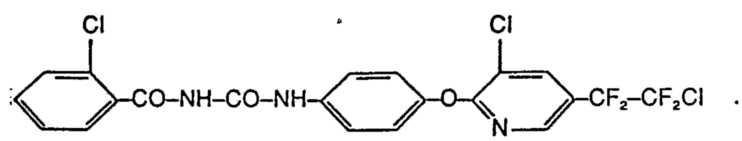

17. Verfahren gemäss Anspruch 4 zur Herstellung der Verbindung der Formel

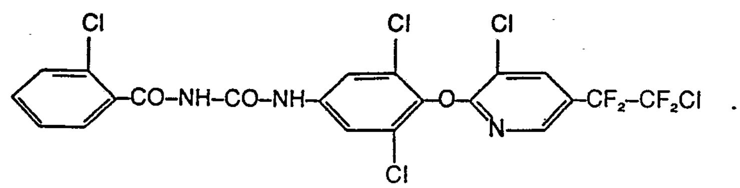

18. Verfahren gemäss Anspruch 4 zur Herstellung der Verbindung der Formel

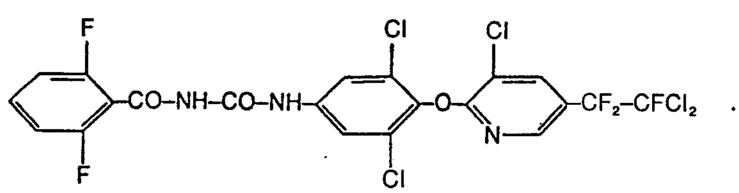

19. Schädlingsbekämpfungsmittel, welches als aktive Komponente eine Verbindung der Formel I gemäss den Ansprüchen 1 bis 18 zusammen mit geeigneten Trägern und/oder anderen Zuschlagstoffen enthält.

20. Verwendung von Verbindungen der Formel I gemäss den Ansprüchen 1 bis 18 zur Bekämpfung von Schädlingen, vorzugsweise von Insekten und Vertretern der Ordnung Akarina.

21. Verwendung gemäss Anspruch 20 als Larvizide und Ovizide zur Bekämpfung pflanzenschädigender Insekten.

22. Verfahren zur Herstellung einer Verbindung der Formel II gemäss Anspruch 1

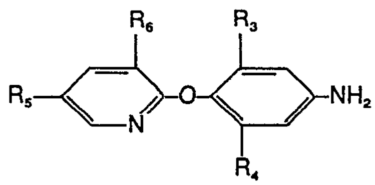 (II)

worin $R_3$ bis $R_6$ die in Anspruch 1 angegebenen Bedeutungen haben, dadurch gekennzeichnet, dass man eine Verbindung der Formel VI

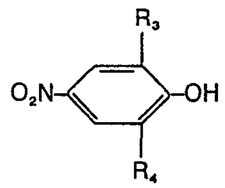 (VI)

mit einer Verbindung der Formel VII

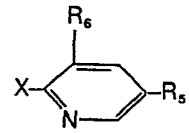 (VII)

umsetzt und die erhaltene Verbindung der Formel VIII

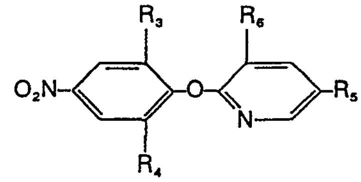 (VIII)

reduziert, wobei in den Formeln VI bis VIII $R_3$ bis $R_6$ die in Anspruch 1 angegebenen Bedeutungen haben und X Halogen, vorzugsweise Chlor, bedeutet.

**Claims for the contracting States: BE, CH, DE, FR, GB, IT, LI, NL**

1. Compounds of the formula I

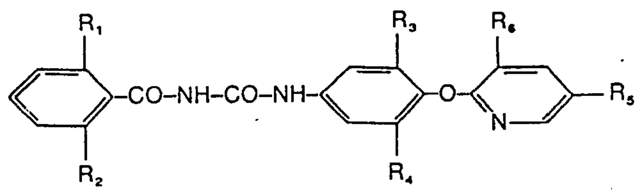 (I),

wherein

$R_1$, $R_2$, $R_3$ and $R_4$ independently of one another are each hydrogen, methyl or halogen,

$R_5$ is the radical $-CHF_2$, or a $C_2$–$C_{10}$-alkyl group which is uniformly or non-uniformly substituted by 1 to 21 halogen atoms, and

$R_6$ is halogen.

2. Compounds of the formula I according to Claim 1, characterised in that

$R_1$, $R_2$, $R_3$ and $R_4$ independently of one another are each hydrogen, methyl, fluorine, chlorine or bromine,

$R_5$ is the radical $-CHF_2$, or a $C_2$–$C_{10}$-alkyl group which is substituted by 1 to 21 fluorine, chlorine or bromine atoms, and

$R_6$ is fluorine, chlorine or bromine.

3. Compounds of the formula I according to Claim 2, characterised in that $R_5$ is the radical $-CHF_2$, or an ethyl group which is uniformly or non-uniformly substituted by 1 to 5 fluorine or chlorine atoms.

4. Compounds of the formula I according to Claim 3, characterised in that $R_5$ is a radical from the group comprising: $-CH_2-CF_3$, $-CF_2-CF_2Cl$, $-CF_2CFCl_2$, $-CCl_2-CCl_3$, $-CF_2-CCl_3$, $-CF_2-CH_3$, $-CCl_2-CH_3$, $-CF_2-CF_3$, $-CH_2-CH_2Cl$, $-CH_2-CHCl_2$ and $-CH_2-CCl_3$.

5. Compounds of the formula I according to Claims 1 to 5, characterised in that $R_3$ and $R_4$ independently of one another are each hydrogen, methyl or chlorine.

6. A compound of the formula I according to Claims 1 to 6, characterised in that $R_6$ is chlorine.

7. A compound according to Claim 4 of the formula

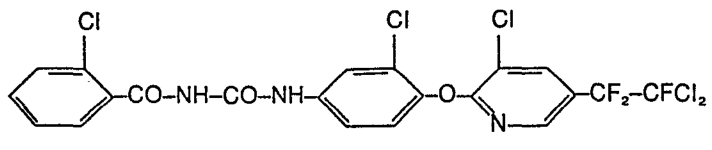 .

8. A compound according to Claim 4 of the formula

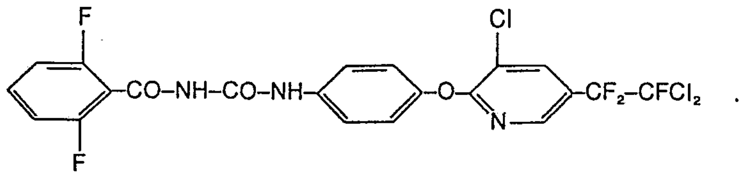

9. A compound according to Claim 4 of the formula

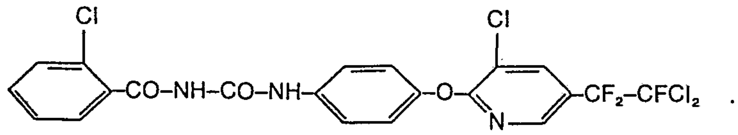

10. A compound according to Claim 4 of the formula

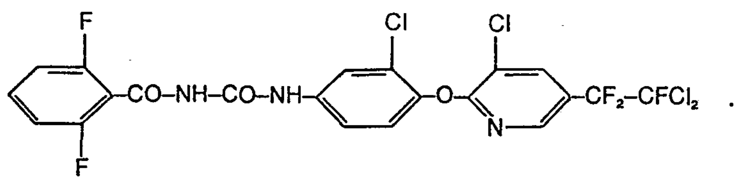

11. A compound according to Claim 4 of the formula

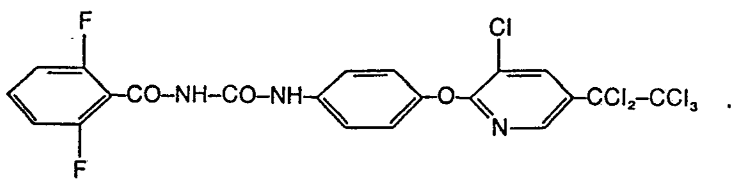

12. A compound according to Claim 4 of the formula

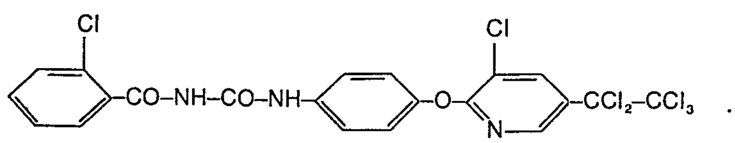

13. A compound according to Claim 3 of the formula

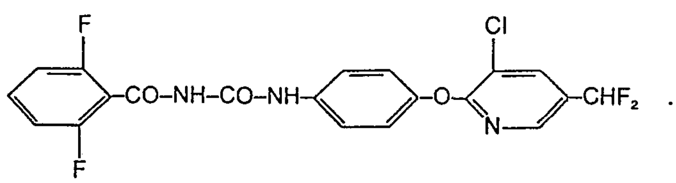

14. A compound according to Claim 3 of the formula

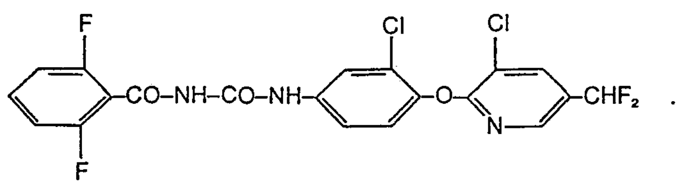

15. A compound according to Claim 4 of the formula

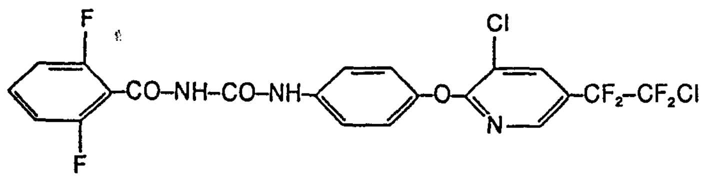

.

16. A compound according to Claim 4 of the formula

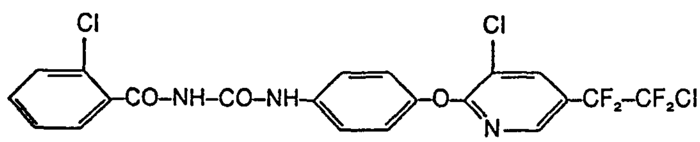

.

17. A compound according to Claim 4 of the formula

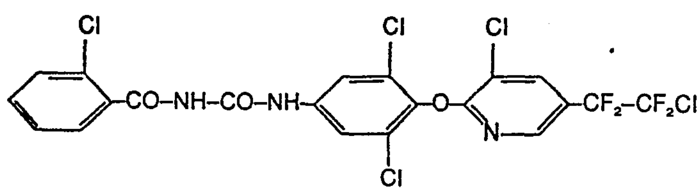

.

18. A compound according to Claim 4 of the formula

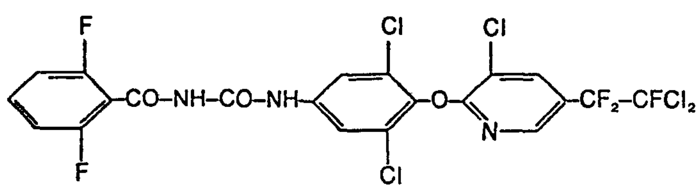

.

19. A process for producing a compound according to Claims 1 to 18, characterised in that

a) a compound of the formula II

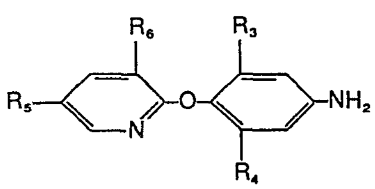

(II)

is reacted with a compound of the formula III

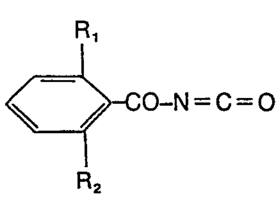

(III)

or

b) a compound of the formula IV

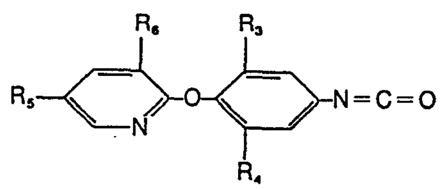

(IV)

is reacted with a compound of the formula V

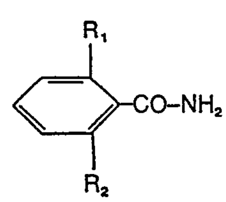

(V).

the radicals $R_1$ to $R_6$ in the formulae II to V having the meanings defined in Claims 1 to 6.

20. A pesticidal composition which contains as active ingredient a compound according to Claims 1 to 19, together with suitable carriers and/or other additives.

21. Use of compounds according to Claims 1 to 18 for combating pests, especially insects and members of the order Acarina.

22. Use according to Claim 21 as larvicides and ovicides for combating insects that damage plants.

23. A compound of the formula II

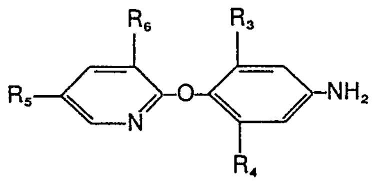 (II)

wherein $R_3$ to $R_6$ have the meanings defined in Claims 1 to 6.

24. A compound of the formula IV

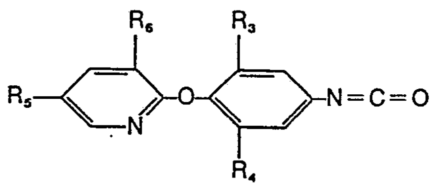 (IV)

wherein $R_3$ to $R_6$ have the meanings defined in Claims 1 to 6.

25. A process for producing a compound of the formula II according to Claim 23, characterised in that a compound of the formula VI

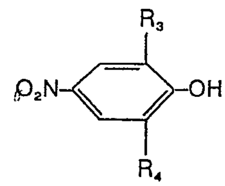 (VI)

is reacted with a compound of the formula VII

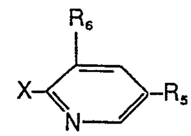 (VII),

and the resulting compound of the formula VIII

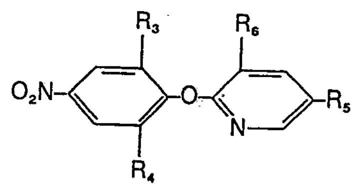 (VIII),

is reduced, $R_3$ to $R_6$ in the formulae VI to VIII having the meanings defined in Claims 1 to 6, and X being halogen, preferably chlorine.

**Claims for the contracting State: AT**

1. A process for producing a compound of the formula I

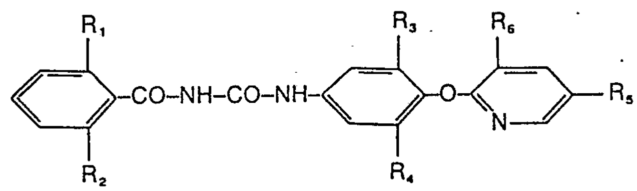 (I),

wherein

$R_1$, $R_2$, $R_3$ and $R_4$ independently of one another are each hydrogen, methyl or halogen,

$R_5$ is the radical $-CHF_2$, or a $C_2$–$C_{10}$-alkyl group which is uniformly or non-uniformly substituted by 1 to 21 halogen atoms, and

$R_6$ is halogen, characterised in that

a) a compound of the formula II

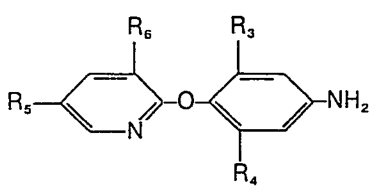 (II)

is reacted with a compound of the formula III

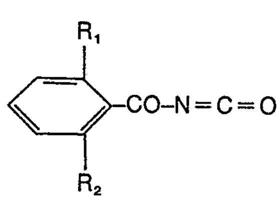 (III)

or

b) a compound of the formula IV

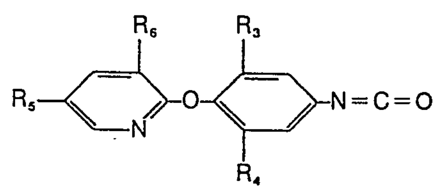 (IV)

is reacted with a compound of the formula V

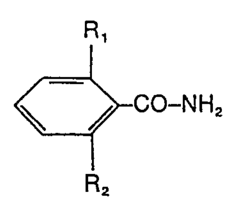 (V).

the radicals $R_1$ to $R_6$ in the formulae II to V having the meanings defined above.

2. A process according to Claim 1 for producing a compound of the formula I, characterised in that

$R_1$, $R_2$, $R_3$ and $R_4$ independently of one another are each hydrogen, methyl, fluorine, chlorine or bromine,

$R_5$ is the radical $-CHF_2$, or a $C_2$–$C_{10}$-alkyl group which is substituted by 1 to 21 fluorine, chlorine or bromine atoms, and

$R_6$ is fluorine, chlorine or bromine.

3. A process according to Claim 2 for producing a compound of the formula I, characterised in that $R_5$ is the radical $-CHF_2$, or an ethyl group which is uniformly or non-uniformly substituted by 1 to 5 fluorine or chlorine atoms.

4. A process according to Claim 3 for producing a compound of the formula I, characterised in that $R_5$ is a radical from the group comprising: $-CH_2-CF_3$, $-CF_2-CF_2Cl$, $-CF_2CFCl_2$, $-CCl_2-CCl_3$, $-CF_2-CCl_3$, $-CF_2-CH_3$, $-CCl_2-CH_3$, $-CF_2-CF_3$, $-CH_2-CH_2Cl$, $-CH_2-CHCl_2$ and $-CH_2-CCl_3$.

5. A process according to Claims 1 to 4 for producing a compound of the formula I, characterised in that $R_3$ and $R_4$ independently of one another are each hydrogen, methyl or chlorine.

6. A process according to Claims 1 to 5 for producing a compound of the formula I, characterised in that $R_6$ is chlorine.

7. A process according to Claim 4 for producing a compound of the formula

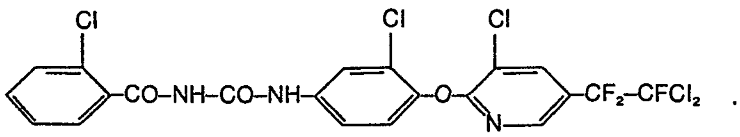

8. A process according to Claim 4 for producing a compound of the formula

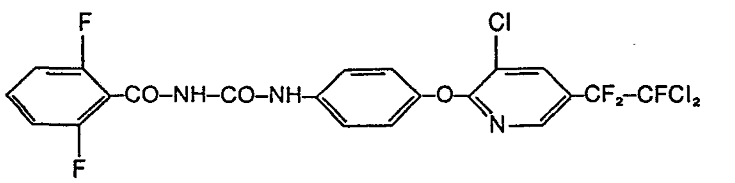

9. A process according to Claim 4 for producing a compound of the formula

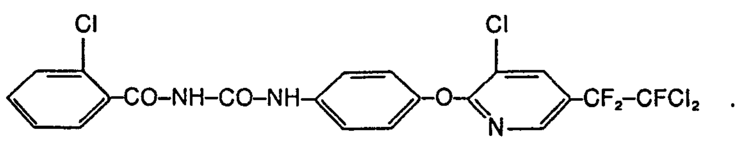

10. A process according to Claim 4 for producing a compound of the formula

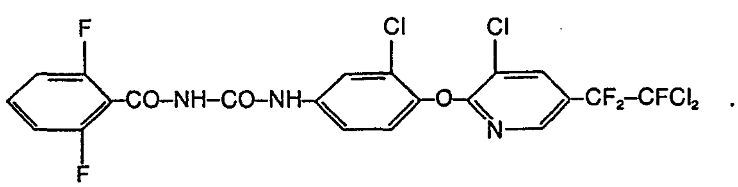

11. A process according to Claim 4 for producing a compound of the formula

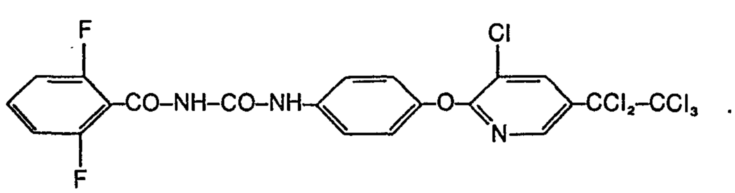

12. A process according to Claim 4 for producing a compound of the formula

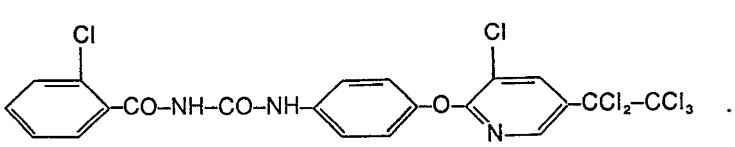

13. A process according to Claim 3 for producing a compound of the formula

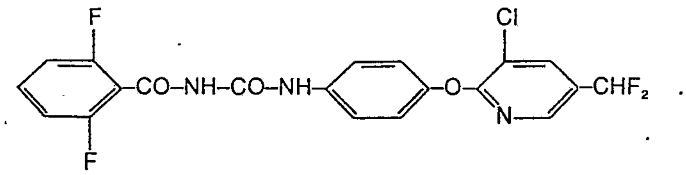

14. A process according to Claim 3 for producing a compound of the formula

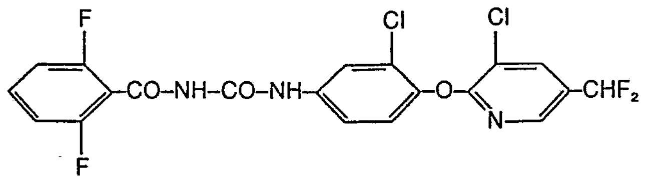

15. A process according to Claim 4 for producing a compound of the formula

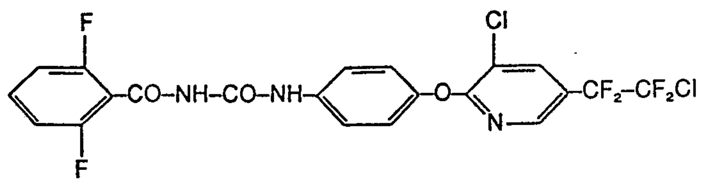

16. A process according to Claim 4 for producing a compound of the formula

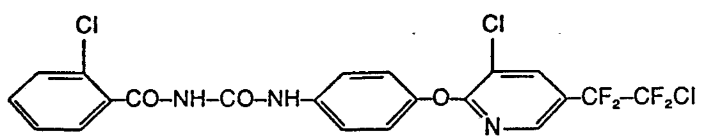

17. A process according to Claim 4 for producing a compound of the formula

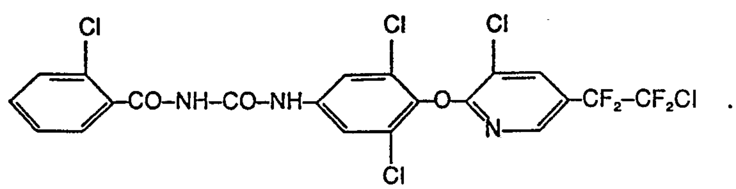

18. A process according to Claim 4 for producing a compound of the formual

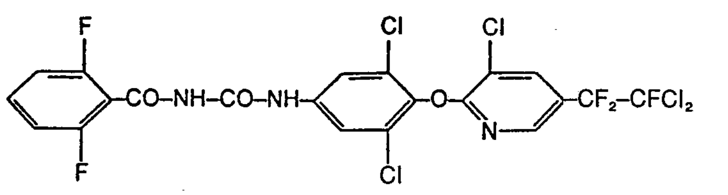

19. A pesticidal composition which contains as active ingredient a compound of the formula I according to Claims 1 to 18, together with suitable carriers and/or other additives.

20. Use of compounds of the formula I according to Claims 1 to 18 for combating pests, especially insects and members of the order Acarina.

21. Use according to Claim 20 as larvicides and ovicides for combating insects that damage plants.

22. A process for producing a compound of the formula II according to claim 1

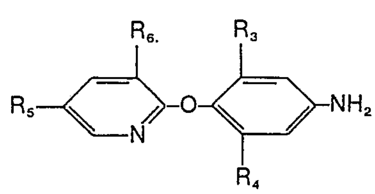

(II),

wherein $R_3$ to $R_6$ have the meanings defined in Claim 1, characterised in that a compound of the formula VI

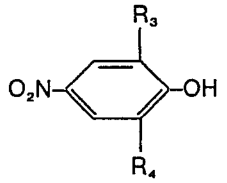

(VI)

is reacted with a compound of the formula VII

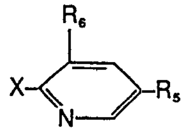

(VII),

and the resulting compound of the formula VIII

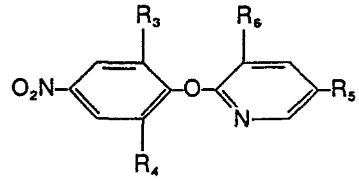

(VIII)

is reduced, $R_3$ to $R_6$ in the formulae VI to VIII having the meanings defined in Claim 1, and X being halogen, preferably chlorine.

**Revendications pour les Etats contractants: BE, CH, DE, FR, GB, IT, LI, NL**

1. Composés de formule I

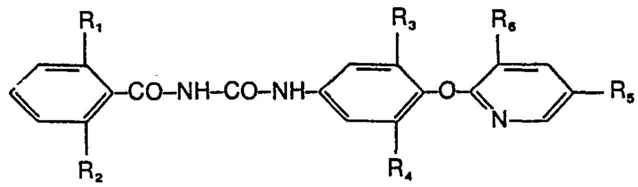

(I),

dans laquelle

$R_1$, $R_2$, $R_3$ et $R_4$ indépendamment l'un de l'autre représentent un hydrogène, méthyle ou halogène;

$R_5$ est le reste $-CHF_2$ ou un reste alkyle $C_2$–$C_{10}$ substitué de manière homogène ou non avec 1 à 21 atomes d'halogène,

et

$R_6$ est un halogène.

2. Composés de formule I selon la revendication 1 caractérisés en ce que

$R_1$, $R_2$, $R_3$ et $R_4$ indépendamment l'un de l'autre représentent un hydrogène, méthyle, fluor, chlore ou brome;

$R_5$ est le reste $-CHF_2$ ou un reste alkyle $C_2$–$C_{10}$ substitué par 1 à 21 atomes de fluor, chlore ou brome et

$R_6$ est le fluor, chlore ou brome.

3. Composés de formule I selon la revendication 2, caractérisés en ce que $R_5$ est le reste $-CHF_2$ ou un reste éthyle substitué de manière homogène ou non avec 1 à 5 atomes de fluor ou de chlore.

4. Composés de formule I selon la revendication 3 caractérisés en ce que $R_5$ est l'un des restes $-CH_2-CF_3$, $-CF_2-CF_2Cl$, $-CF_2CFCl_2$, $-CCl_2-CCl_3$, $-CF_2-CCl_3$, $-CF_2-CH_3$, $-CCl_2-CH_3$, $-CF_2-CF_3$, $CH_2-CH_2Cl$, $-CH_2-CHCl_2$ ou $-CH_2-CCl_3$.

5. Composés de formule I selon les revendications 1 à 5, caractérisés en ce que $R_3$ et $R_4$ indépendamment l'un de l'autre représentent un hydrogène, méthyle ou chlore.

6. Composés de formule I selon les revendications 1 à 6, caractérisés en ce que $R_6$ est le chlore.

7. Composé selon la revendication 4 de formule

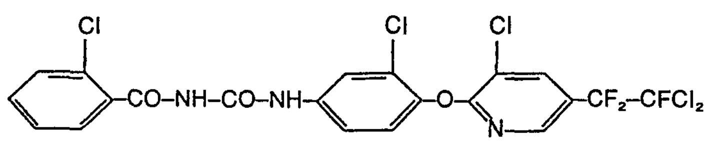

.

8. Composé selon la revendication 4 de formule

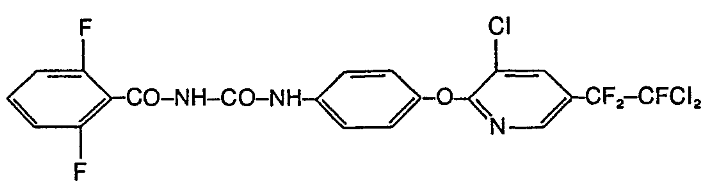

.

9. Composé selon la revendication 4 de formule

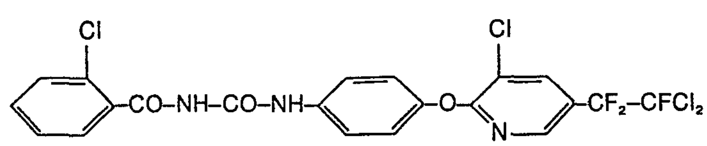

.

10. Composé selon la revendication 4 de formule

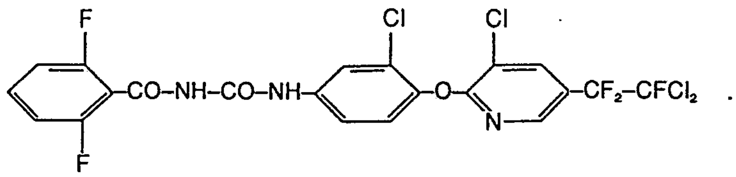

11. Composé selon la revendication 4 de formule

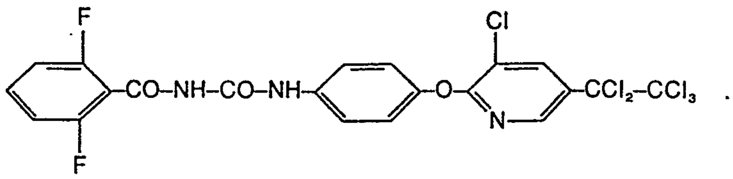

12. Composé selon la revendication 4 de formule

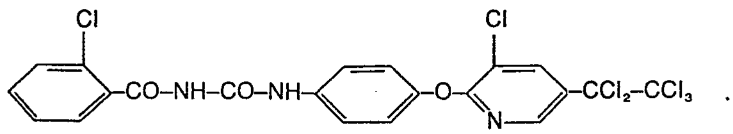

13. Composé selon la revendication 4 de formule

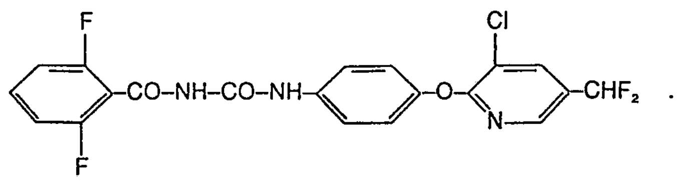

14. Composé selon la revendication 4 de formule

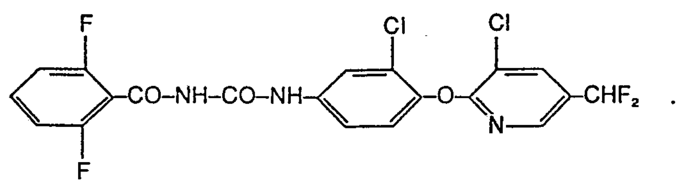

15. Composé selon la revendication 4 de formule

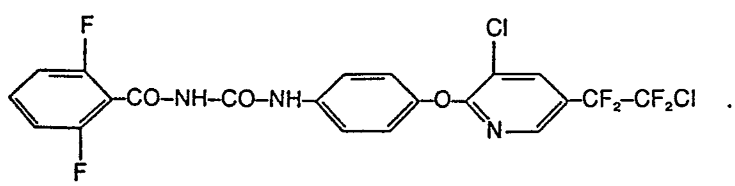

16. Composé selon la revendication 4 de formule

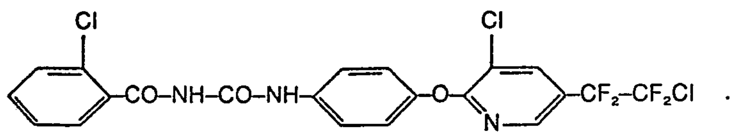

17. Composé selon la revendication 4 de formule

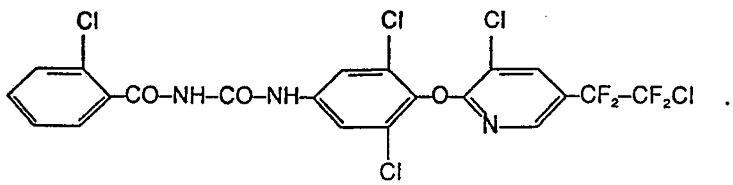

18. Composé selon la revendication 4 de formule

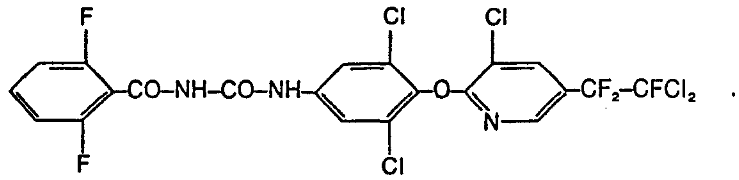

19. Procédé de préparation d'un composé selon les revendications 1 à 18, caractérisé en ce qu'on fait réagir

a) un composé de formule II

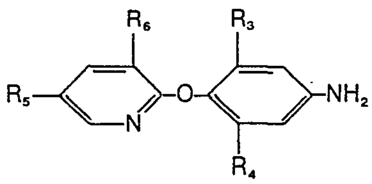 (II)

avec un composé de formule III

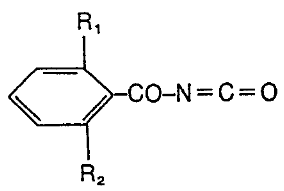 (III)

ou

b) un composé de formule IV

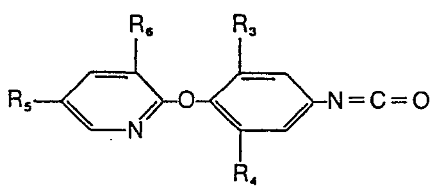 (IV)

avec un composé de formule V

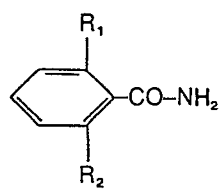 (V)

dans lesquels dans les formules II à V les restes $R_1$ à $R_6$ ont les significations données dans les revendications 1 à 6.

20. Moyen de lutte anti-parasitaire, qui contient comme composants actifs un composé selon les revendications 1 à 19 ainsi que des véhicules appropriés et/ou d'autres additifs.

21. Utilisation de composés selon les revendications 1 à 18 pour lutter contre des parasites, de préférence des insectes et des représentants de l'ordre acarien.

22. Utilisation selon la revendication 21 comme larvicides et ovicides pour lutter contre les insectes nuisibles aux plantes.

23. Composé de formule II

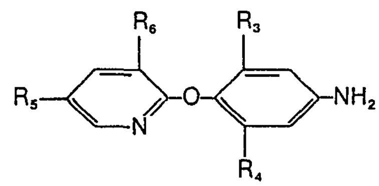 (II)

dans laquelle $R_3$ à $R_6$ ont les significations données dans les revendications 1 à 6.

24. Composé de formule IV

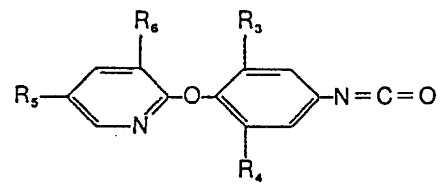 (IV)

dans laquelle $R_3$ à $R_6$ ont les significations données dans les revendications 1 à 6.

25. Procédé de préparation d'un composé de formule II selon la revendication 23, caractérisé en ce qu'on fait réagir un composé de formule VI

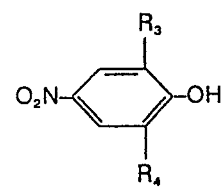 (VI)

avec un composé de formule VII

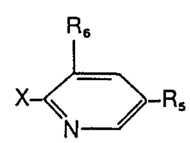 (VII)

et qu'on réduit le composé obtenu de formule VIII

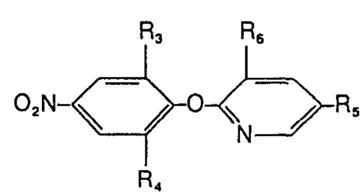 (VIII)

dans lesquels dans les formules VI à VIII $R_3$ à $R_6$ ont les significations données dans les revendications 1 à 6 et X est un halogène, de préférence le chlore.

**Revendications pour l'Etat contractant AT**

1. Procédé de préparation d'un composé de formule I

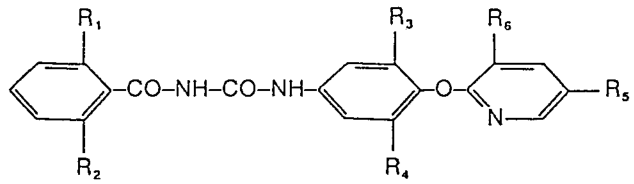

(I)

dans laquelle

$R_1$, $R_2$, $R_3$ et $R_4$ indépendamment l'un de l'autre représentent un hydrogène, méthyle ou halogène;

$R_5$ est le reste $-CHF_2$ ou un reste alkyle $C_2$–$C_{10}$ substitué de manière homogène ou non avec 1 à 21 atomes d'halogène

et

$R_6$ est un halogène

caractérisé en ce qu'on fait réagir

a) un composé de formule II

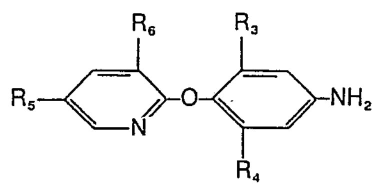

(II)

avec un composé de formule III

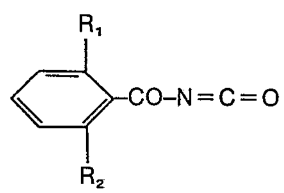

(III)

ou

b) un composé de formule IV

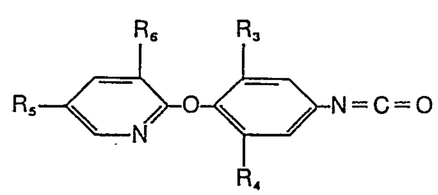

(IV)

avec un composé de formule V

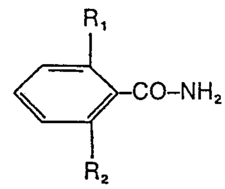

(V)

dans lesquels, dans les formules II à V les restes $R_1$ à $R_6$ ont les significations données plus haut.

2. Procédé selon la revendication 1 pour préparer un composé de formule I, caractérisé en ce que

$R_1$, $R_2$, $R_3$ et $R_4$ indépendamment l'un de l'autre représentent un hydrogène, méthyle, fluor, chlore ou brome;

$R_5$ est le reste $-CHF_2$ ou un reste alkyle $C_2$–$C_{10}$ substitué par 1 à 21 atomes de fluor, chlore ou brome et

$R_6$ est le fluor, chlore ou brome.

3. Procédé selon la revendication 2 pour préparer un composé de formule I caractérisé en ce que $R_5$ est le reste $-CHF_2$ ou un reste éthyle substitué de manière homogène ou non avec 1 à 5 atomes de fluor ou de chlore.

4. Procédé selon la revendication 3 pour préparer un composé de formule I caractérisé en ce que $R_5$ est l'un des restes $-CH_2-CF_3$, $-CF_2-CF_2Cl$, $-CF_2CFCl_2$, $-CCl_2-CCl_3$, $-CF_2-CCl_3$, $-CF_2-CH_3$, $-CCl_2-CH_3$, $-CF_2-CF_3$, $CH_2-CH_2Cl$, $-CH_2-CHCl_2$ ou $-CH_2-CCl_3$.

5. Procédé selon les revendications 1 à 4 pour préparer un composé de formule I, caractérisé en ce que $R_3$ et $R_4$ indépendamment l'un de l'autre représentent un hydrogène, méthyle ou chlore.

6. Procédé selon les revendications 1 à 5 pour préparer un composé de formule I, caractérisé en ce que $R_6$ est le chlore.

7. Procédé selon la revendication 4 pour préparer le composé de formule

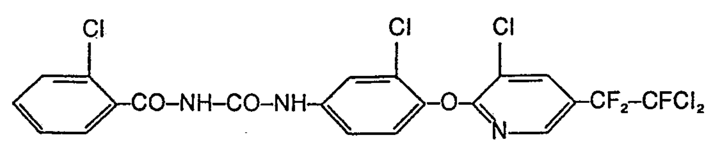 .

8. Procédé selon la revendication 4 pour préparer le composé de formule

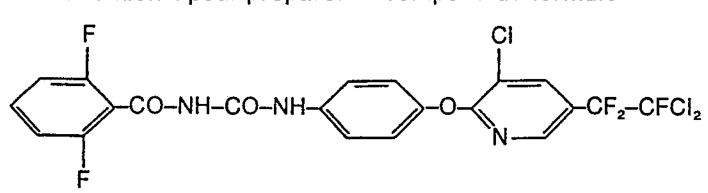 .

9. Procédé selon la revendication 4 pour préparer le composé de formule

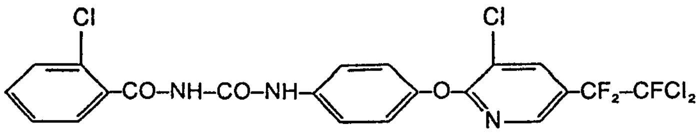 .

10. Procédé selon la revendication 4 pour préparer le composé de formule

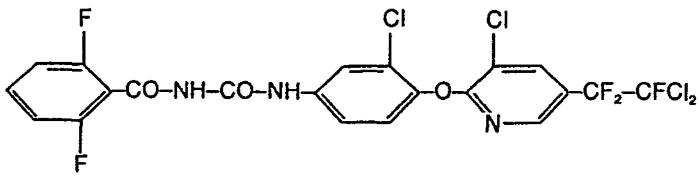 .

11. Procédé selon la revendication 4 pour préparer le composé de formule

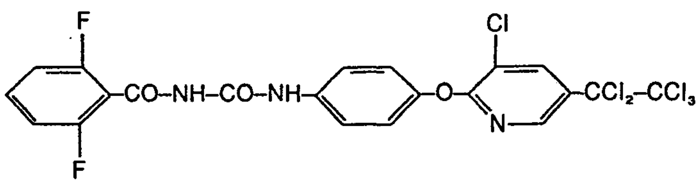 .

12. Procédé selon la revendication 4 pour préparer le composé de formule

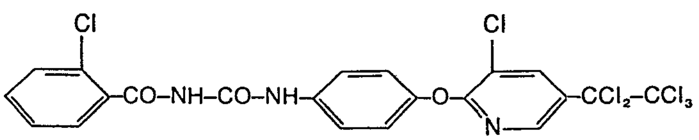 .

13. Procédé selon la revendication 4 pour préparer le composé de formule

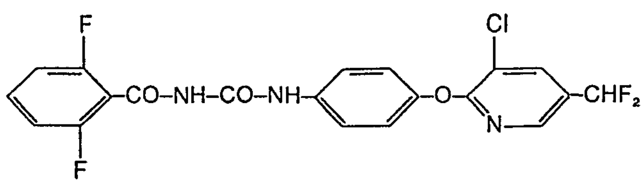 .

14. Procédé selon la revendication 4 pour préparer le composé de formule

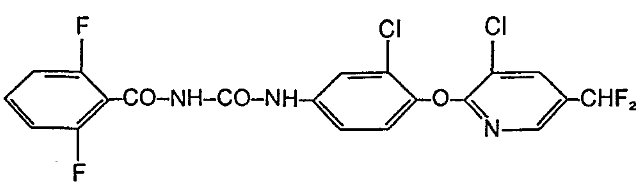 .

15. Procédé selon la revendication 4 pour préparer le composé de formule

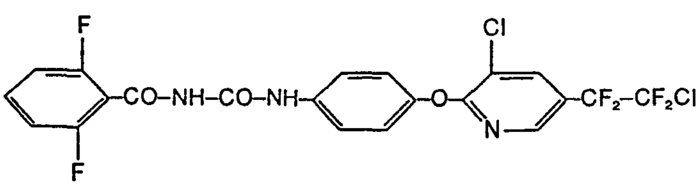 .

16. Procédé selon la revendication 4 pour préparer le composé de formule

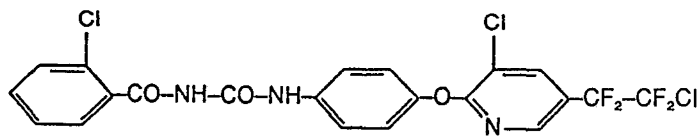 .

17. Procédé selon la revendication 4 pour préparer le composé de formule

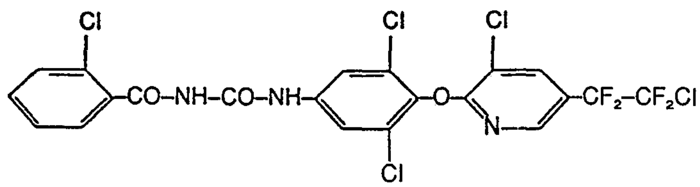 .

18. Procédé selon la revendication 4 pour préparer le composé de formule

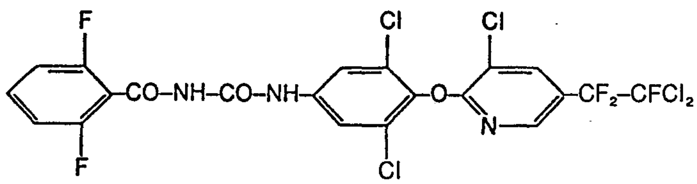 .

19. Moyen de lutte anti-parasitaire, qui contient comme composants actifs un composé de formule I selon les revendications 1 à 18 ainsi que des véhicules appropriés et/ou d'autres additifs.

20. Utilisation de composés de formule I selon les revendications 1 à 18 pour lutter contre des parasites, de préférence des insectes et des représentants de l'ordre acarien.

21. Utilisation selon la revendication 20 comme larvicides et ovicides pour lutter contre les insectes nuisibles aux plantes.

22. Procédé de préparation d'un composé de formule II selon la revendication 1

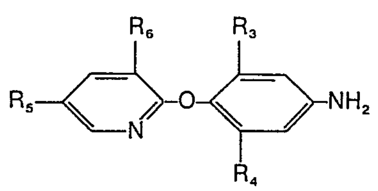 (II)

dans laquelle $R_3$ à $R_6$ ont les significations données dans la revendication 1, caractérisé en ce qu'on fait réagir un composé de formule VI

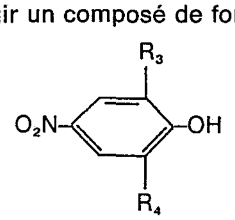 (VI)

avec un composé de formule VII

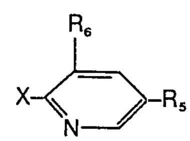 (VII)

et qu'on réduit le composé obtenu de formule VIII

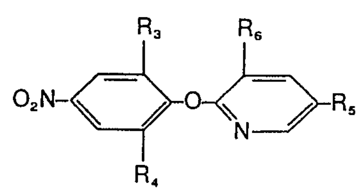 (VIII)

dans lesquels dans les formules VI à VIII $R_3$ à $R_6$ ont les significations données dans la revendication 1 et X est un halogène, de préférence le chlore.